# EUROPEAN PATENT APPLICATION

(11) **EP 1 508 273 A1**
(43) Date of publication of application: **23.02.2005**
(21) Application number: 03728136.7
(22) Date of filing: 26.05.2003
(51) Int. Cl.: A01K 67/027, C12N 15/09, G01N 33/53

(54) **ADIPONECTIN-KNOUCOUT NONHUMAN ANIMAL**

(30) Priority: 24.05.2002 JP 2002151045
(71) Applicant: Japan Science and Technology Agency, Kawaguchi-shi, Saitama 332-0012 (JP)
(72) Inventor: KADOWAKI, Takashi, KAWASAKI-SHI, Kanagawa 215-0000 (JP); KUBOTA, Naoto, TAITO-KU, Tokyo 110-0008 (JP); TERAUCHI, Yasuo, MINATO-KU, Tokyo 108-0073 (JP); YAMAUCHI, Toshimasa, BUNKYO-KU, Tokyo 113-0023 (JP); NODA, Tetsuo, BUNKYO-KU, Tokyo 112-0003 (JP)
(74) Representative: Hartz, Nikolai F., Dr.
(86) International application number: PCT/JP2003/006519
(87) International publication number: WO 2003/099003

(57) **Abstract**

The animals of the present invention enable elucidation of the onset mechanisms of obesity, diabetes, and arteriosclerosis. Moreover, they are useful for screening preventive and therapeutic drugs for treating any of these diseases.

SEQUENCE LISTING

<110> Japan science and Technology Corporation

<120> Adiponectin gene nock-out animal

<130> JST0012

<140>
<141>

<150> JP 2002-151045
<151> 2002-5-24

<160> 2

<170> PatentIn Ver. 2.1

<210> 1
<211> 1276
<212> DNA
<213> Mus musculus

<220>
<221> CDS
<222> (46)..(789)

<400> 1

ctctaaagat tgtcagtgga tctgacgaca ccaaaagggc tcagg atg cta ctg

ttg 57

Met Leu Leu Leu

1

caa gct ctc ctg ttc ctc tta atc ctg ccc agt cat gcc gaa gat

gac 105 Gln Ala Leu Leu Phe Leu Leu Ile Leu Pro Ser His

Ala Glu Asp Asp

5 10 15 20

gtt act aca act gaa gag cta gct cct gct ttg gtc cct cca ccc

aag 153 Val Thr Thr Thr Glu Glu Leu Ala Pro Ala Leu Val

Pro Pro Pro Lys

25 30 35

gga act tgt gca ggt tgg atg gca ggc atc cca gga cat cct ggc

cac 201 Gly Thr Cys Ala Gly Trp Met Ala Gly Ile Pro Gly

His Pro Gly His

40 45 50

aat ggc aca cca ggc cgt gat ggc aga gat ggc act cct gga gag

aag 249 Asn Gly Thr Pro Gly Arg Asp Gly Arg Asp Gly Thr

Pro Gly Glu Lys

55 60 65

gga gag aaa gga gat gca ggt ctt ctt ggt cct aag ggt gag aca

gga   297   Gly Glu Lys Gly Asp Ala Gly Leu Leu Gly Pro Lys
Gly Glu Thr Gly
    70                  75                  80

gat gtt gga atg aca gga gct gaa ggg cca cgg ggc ttc ccc gga
acc   345   Asp Val Gly Met Thr Gly Ala Glu Gly Pro Arg Gly
Phe Pro Gly Thr
 85                 90                  95                  100

cct ggc agg aaa gga gag cct gga gaa gcc gct tat atg tat cgc
tca   393   Pro Gly Arg Lys Gly Glu Pro Gly Glu Ala Ala Tyr
Met Tyr Arg Ser
                105                 110                 115

gcg ttc agt gtg ggg ctg gag acc cgc gtc act gtt ccc aat gta
ccc   441   Ala Phe Ser Val Gly Leu Glu Thr Arg Val Thr Val
Pro Asn Val Pro
            120                 125                 130

att cgc ttt act aag atc ttc tac aac caa cag aat cat tat gac
ggc   489   Ile Arg Phe Thr Lys Ile Phe Tyr Asn Gln Gln Asn
His Tyr Asp Gly
        135                 140                 145

agc act ggc aag ttc tac tgc aac att ccg gga ctc tac tac ttc
tct   537   Ser Thr Gly Lys Phe Tyr Cys Asn Ile Pro Gly Leu
Tyr Tyr Phe Ser

150 155 160

tac cac atc acg gtg tac atg aaa gat gtg aag gtg agc ctc ttc
aag 585 Tyr His Ile Thr Val Tyr Met Lys Asp Val Lys Val
Ser Leu Phe Lys
165 170 175 180

aag gac aag gcc gtt ctc ttc acc tac gac cag tat cag gaa aag
aat 633 Lys Asp Lys Ala Val Leu Phe Thr Tyr Asp Gln Tyr
Gln Glu Lys Asn
185 190 195

gtg gac cag gcc tct ggc tct gtg ctc ctc cat ctg gag gtg gga
gac 681 Val Asp Gln Ala Ser Gly Ser Val Leu Leu His Leu
Glu Val Gly Asp
200 205 210

caa gtc tgg ctc cag gtg tat ggg gat ggg gac cac aat gga ctc
tat 729 Gln Val Trp Leu Gln Val Tyr Gly Asp Gly Asp His
Asn Gly Leu Tyr
215 220 225

gca gat aac gtc aac gac tct aca ttt act ggc ttt ctt ctc tac
cat 777 Ala Asp Asn Val Asn Asp Ser Thr Phe Thr Gly Phe
Leu Leu Tyr His
230 235 240

gat acc aac tga ctgcaactac ccatagccca tacaccagga gaatcatgga
829 Asp Thr Asn
245

acagtcgaca cactttcagc ttagtttgag agattgattt tattgcttag
tttgagagtc 889

ctgagtatta tccacacgtg tactcacttg ttcattaaac gactttataa
aaaataattt 949

gtgttcctag tccagaaaaa aaggcactcc ctggtctcca cgactcttac
atggtagcaa 1009

taacagaatg aaaatcacat ttggtatggg ggcttcacaa tattcgcatg
actgtctgga 1069

agtagaccat gctatttttc tgctcactgt acacaaatat tgttcacata
aaccctataa 1129

tgtaaatatg aaatacagtg attactcttc tcacaggctg agtgtatgaa
tgtctaaaga 1189

cccataagta ttaaagtggt agggataaat tggaaaaaaa aaaaaaaaaa
aagaaaaact 1249

ttagagcaca ctggcggccg ttactag 1276

## Description

### Technical Field

The present invention relates to an animal model of obesity and/or diabetes, and to a non-human animal which is useful as an animal model of arteriosclerosis.

### Background Art

Of Japanese patients suffering from diabetes or obesity, estimated seven million patients suffer from diabetes, and the number thereof increases as ever. Common type-2 diabetes, which accounts for major part of diabetes, and obesity as well, are pathological conditions which are developed when a plurality of causal genes and environmental factors, such as those related to one's lifestyle, work together. The primary cause for the increase in diabetes/obesity cases is considered to be tendency of elevated insulin resistance, which is caused by changes in the lifestyle, including westernization in diet, in particular high fat diet, and less physical exercise. Thus, there is urgent need for identifying causal genes responsible for type-2 diabetes/obesity, and for elucidating the molecular mechanism which prompts insulin resistance and eventually induces syndrome X or arteriosclerosis, to thereby establish a radical preventive or therapeutic treatment for lifestyle-related diseases.

Meanwhile, arteriosclerosis is also considered as one type of lifestyle-related disease, and may become a crucial cause for cerebral hemorrhage, cerebral infarction, myocardial infarction, nephrosclerosis, etc. The major condition of arteriosclerosis is thickening of the arterial intima, and therefore, development of a drug capable of directly preventing the same has been desired.

In developing such a drug, excellent animal models are indispensable.

Accordingly, an object of the present invention is to provide an animal model for lifestyle-related diseases.

### Disclosure of the Invention

Under the above circumstances, the present inventors have performed research, focusing on adiponectin isolated from human adipose tissue, and have found that adiponectin-gene-deficient (hereinafter may be referred to as "adiponectin gene knockout") non-human animals exhibit not only significant insulin resistance but also considerable thickening of arterial intima, and thus are useful as animal models of obesity, diabetes, and arteriosclerosis. The present invention has been accomplished on the basis of these findings.

Accordingly, the present invention provides a non-human animal lacking the functions of adiponectin.

Also, the present invention provides an animal model of obesity, diabetes, and/or arteriosclerosis, which is established with a non-human animal lacking the functions of adiponectin.

Moreover, the present invention provides a method for screening preventive and therapeutic agents for obesity, diabetes, and/or arteriosclerosis, which method is characterized by administering a test drug to a non-human animal lacking the functions of adiponectin.

### Brief Description of the Drawings

Fig. 1 shows the gene targeting strategy used to ablate the adiponectin gene. Top: restriction enzyme map of the mouse adiponectin gene. Middle: adiponectin gene targeting vector. Bottom: deduced targeting allele.
Fig. 2 shows the results of Southern blotting performed on DNA samples derived from ES cells and digested with SpeI and EoRV. The 17-kb bands indicate a wild-type allele, and the 10.5-kb bands indicate a mutated allele.
Fig. 3 shows the results of Southern blotting performed on DNA samples derived from different types of mice; i.e., wild-type, hetero-deficient (adipo +/-), and homo-deficient (adipo -/-), and digested with SpeI and EoRV. The 17-kb bands indicate a wild-type allele, and the 10.5-kb bands indicate a mutated allele.
Fig. 4 shows the results of Northern blotting performed on white adipose tissue samples of different types of mice; i.e., wild-type, hetero-deficient (adipo +/-), and homo-deficient (adipo -/-).
Fig. 5 is a graph showing the adiponectin level of blood samples drawn from different types of mice; i.e., wild-type, hetero-deficient (adipo +/-), and homo-deficient (adipo -/-). **P<0.01.
Fig. 6 is a graph showing the leptin level of blood samples drawn from different types of mice; i.e., wild-type, hetero-deficient (adipo +/-), and homo-deficient (adipo -/-).
Fig. 7 is a graph showing the body weight of different types of mice; i.e., wild-type, hetero-deficient (adipo +/-), and homo-deficient (adipo -/-), as weighed at 6 weeks of age.
Fig. 8 is a graph showing the results from an insulin tolerance test performed on wild-type mice and hetero-deficient mice (adipo +/-), as measured at 6 weeks of age. *P<0.05.
Fig. 9 is a graph showing the results from a glucose tolerance test performed on wild-type mice and hetero-deficient mice (adipo +/-), as measured at 6 weeks of age [(1): blood sugar level, (2): insulin level]. *P<0.05.
Fig. 10 is a graph showing the results from a glucose tolerance test performed on wild-type mice and hetero-deficient mice (adipo +/-), as measured after 10 weeks of loading with high-fat diet [(1): blood sugar level, (2): insulin level]. *P<0.05, **P<0.01.
Fig. 11 is a graph showing the results from an insulin tolerance test performed on wild-type mice and homo-deficient mice (adipo -/-), as measured at 6 weeks of age. *P<0.05, **P<0.01.
Fig. 12 is a graph showing the results from a glucose tolerance test performed on wild-type mice and hetero-deficient mice (adipo +/-), as measured at 6 weeks of age [(1): blood sugar level, (2): insulin level]. *P<0.05, **P<0.01.
Fig. 13 shows levels, in blood of free fatty acid (FFA), neutral fat (TG), and total cholesterol (TC), as determined in wild-type mice and hetero-deficient mice (adipo +/-).
Fig. 14 shows levels, in blood of free fatty acid (FFA), neutral fat (TG), and total cholesterol (TC), as determined in wild-type mice and homo-deficient mice (adipo -/-).
Fig. 15 shows the inner diameter of blood vessel of wild-type and hetero-deficient (adipo +/-) mice, as measured two weeks after the mice underwent cuff placement.
Fig. 16 shows the degree of intimal thickening of wild-type and hetero-deficient (adipo +/-) mice, as measured two weeks after the mice underwent cuff placement.
Fig. 17 shows the degree of medial thickening of wild-type and hetero-deficient (adipo +/-) mice, as measured two weeks after the mice underwent cuff placement.
Fig. 18 shows the intima/media ratio of wild-type and hetero-deficient (adipo +/-) mice, as measured two weeks after the mice underwent cuff placement.

### Best Modes for Carrying Out the Invention

The non-human animals of the present invention lacking the functions of adiponectin are specifically defined as adiponectin gene knockout non-human animals.

Adiponectin has already been cloned (Maeda, K. et al., Biochem. Biophys. Res. Commun. 221, 286-296 (1996), Nakano, Y. et al., J. Biochem. (Tokyo) 120, 802-812 (1996)), and is available through known means. SEQ ID NOs: 1 and 2 show the nucleotide sequence and the amino acid sequence of mouse adiponectin.

As used herein, the expression "lacking the functions of adiponectin gene" means that adiponectin, which is the product of the gene, is not produced properly, and includes the case where adiponectin itself is not produced at all and also the case where adiponectin-like protein is produced, the adiponectin-like protein being partially deficient and thus unable to express the functions of intact adiponectin.

In order to obtain an adiponectin gene knockout animal of the present invention, the following procedure is commonly employed. That is, a relevant gene is cloned, then the function of the gene is ablated in vitro, and the resultant gene is returned into an animal to thereby induce homologous recombination with chromosomal adiponectin gene, followed by disruption of the chromosomal adiponectin gene, whereby loss of function of the relevant gene of that individual animal or the relevant genes of its offspring is introduced.

In order to achieve the loss of function of a gene, mutation may be artificially introduced to the gene so as to disrupt the gene. For example, at least a portion of the promoter region and/or the coding region may be deleted or another gene may be inserted or substituted.

In the present invention, the non-human animal may be any animal so long as it is deficient in adiponectin gene function, and includes both heterozygous and homozygous animals in terms of adiponectin gene knockout mutation. Also, no particular limitation is imposed on the animal to be employed, and any animal which falls within the category of mammals, excepting the human, may be employed. Preferred examples of the mammals include guinea pigs, hamsters, mice, rats, rabbits, and pigs. Of these, rodents, particularly mice, are more preferred because they can be easily handled as models representing pathological conditions and have a relatively short life cycle.

In order to transfer a gene into an individual animal so as to cause expression of the gene in the animal or its offspring, any known technique which has hitherto been routinely employed for producing transgenic animals may be used. For example, a host embryo is obtained through a method of transferring gene DNA into a pronuclear phase embryo of a fertilized egg, a method of infecting an early embryo with recombinant retrovirus, or a method of injecting embryonic stem cells (ES cells) which have undergone homologous recombination into blastocysts or 8-cell stage embryos; then is transplanted into an animal to produce offspring; and then crossbred with another individual, to thereby yield F1 heterozygously mutated animals, and further F2 homozygously or hemizygously mutated animals.

Of the above-mentioned methods, the gene transfer method by use of ES cells is suitable for effecting disruption (knockout) of a gene by homologous recombination. This method is preferred because of the advantage that the step of introducing a gene into ES cells and the step of producing a chimeric animal can be performed separately one from the other. The gene transfer method through the employment of ES cells may be performed in accordance with known methods.

A gene transfer method through employment of ES cells will next be described in detail with reference to an example which utilizes mice.

In order to introduce a loss-of-function mutation to mouse adiponectin gene, at least a portion of the promoter region and/or the coding region may be deleted, or another gene may be inserted to any suitable site thereof. Also, so long as such a "loss of gene function" can be attained, the site at which deletion is effected or insertion of another gene is effected may be present in an intron.

When homologous recombination with adiponectin gene is performed, firstly, a DNA fragment (targeting vector) having a DNA sequence so as to knockout the gene is constructed.

Preferably, the gene to be inserted can function as a marker gene for detecting deficiency in adiponectin gene. Examples of such a gene include, but are not limited to, neomycin (neo) resistance gene, which serves as a marker gene for effecting positive selection; and thymidine kinase (tk) gene and diphtheria toxin A fragment (DT-A) gene, which serve as marker genes for effecting negative selection. It should be noted that the neomycin resistance gene enables selection of a gene of interest, through use of a neomycin analogue G418.

In a preferred gene targeting strategy, there may be performed either of the following two methods. One method employs, as a targeting vector, a "substituted" vector prepared by introducing, as a substituent, a positive selection marker onto the target gene. Another method employs an "inserted" vector, which is obtained by inserting, to the upstream of the target gene, a non-homologous region serving as a backbone of a vector containing a selection marker, to thereby inhibit expression of the gene. The insertion may be performed in vitro through a conventional DNA recombination technique.

Next, homologous recombination is carried out between the thus-obtained targeting vector and the adiponectin gene contained in ES cells. In order to carry out homologous recombination through transfer of DNA into ES cells, conventional electroporation may be used. During the process of homologous recombination, recombination occurs between the DNA of adiponectin gene from ES cells and a corresponding region of the DNA for homologous recombination; whereby the marker gene that has been inserted into the DNA for homologous recombination is inserted into the genomic adiponectin gene of ES cells. As a result, ES cells will come to lose the functions of adiponectin gene, and at the same time, come to have a marker gene. This marker gene selection mechanism enables selection of ES cells lacking the functions of CBP gene.

Subsequently, the thus-obtained ES cells are injected into embryos of a host, such as mouse blastocysts, and the resultant embryos are transplanted to the uterine horns of pseudopregnant mice, whereby chimeric mice are produced. Crossbreeding of the chimeric mice with mice of a suitable line, F1 heterozygous offspring can be obtained. If the germ cells of the chimeric mice are derived from the homologous recombinants, or in other words, derived from adiponectin gene knockout ES cells, mice deficient in adiponectin gene function can be obtained. Also, when one of the obtained heterozygous animals is crossbred with another one of the obtained heterozygous animals, homozygously (-/-) mutated animals may be identified among the offspring animals.

The question as to whether or not the animal is deficient in adiponectin gene, or whether the gene is heterozygously (+/-) deficient or homozygously (-/-) deficient can be verified through Southern blotting or PCR, which is performed on DNA extracted from the tail after ablactation of the animal.

Breeding the animals of the present invention does not require any special methodology, and the animals can be raised in a manner similar to that employed for normal animals.

The thus-produced animals lacking adiponectin functions; in particular, adiponectin heterozygous (+/-) knockout animals, exhibit the following traits.
(1) As compared with wild-type counterparts, adiponectin-knockout animals show a significant insulin resistance, despite of blood sugar level being elevated in a glucose tolerance test. Also, since the glucose tolerance test reveals elevated blood sugar levels in both cases of fasting conditions and glucose loading, the adiponectin-knockout animals have impaired glucose tolerance.
(2) Adiponectin knockout animals are comparable with wild-type counterparts in terms of blood free fatty acid total cholesterol level. However, homozygous (-/-) knockout animals present high levels of neutral fat.
(3) Adiponectin knockout animals present significant intimal thickening in response to arterial cuff-induced injury, manifesting clear arteriosclerosis conditions.

Accordingly, the adiponectin knockout animals of the present invention are useful as animal models of obesity and/or diabetes, in particular obesity and/or type 2 diabetes. Moreover, they are useful as animal models of arteriosclerosis.

Screening of obesity and/or diabetes preventive and therapeutic agents or arteriosclerosis preventive and therapeutic agents by the employment of animals of the present invention which are deficient in adiponectin functions may be performed as follows: A test drug is administered to animals lacking adiponectin functions, and the above-described traits inherent to the animals deficient in adiponectin functions are investigated, or alternatively, changes in expression level of a gene unique to animals deficient in adiponectin functions may be monitored. In the latter case, changes in the gene expression level can be conveniently performed through analysis by use of DNA chip technologies or similar techniques.

### Examples

The present invention will next be described in more detail by way of examples, which should not be construed as limiting the invention thereto.

### A. Method

### (1) Generation of knockout mice

Screening of a 129/Sv mouse genomic library was performed by use of adiponectin cDNA as a probe, whereby a plurality of clones each harboring the adiponectin gene were cloned. A targeting vector was constructed by substituting a neomycin resistance gene for a stretch spanning from the site directly adjacent to the translation initiation point to the translation termination point, and ES cells were transfected with the thus-prepared targeting vector. Screening by Southern blotting confirmed 5 clones of homologous recombinants. Through microinjection, chimeric mice were generated, and the mice were crossbred with BI/6, to produce F1, and further F2 mice.

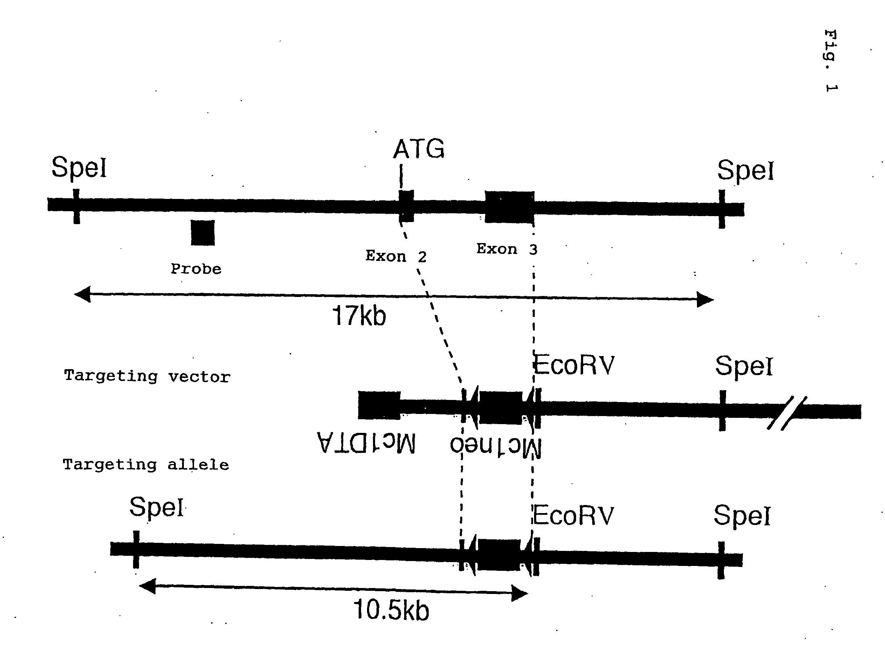

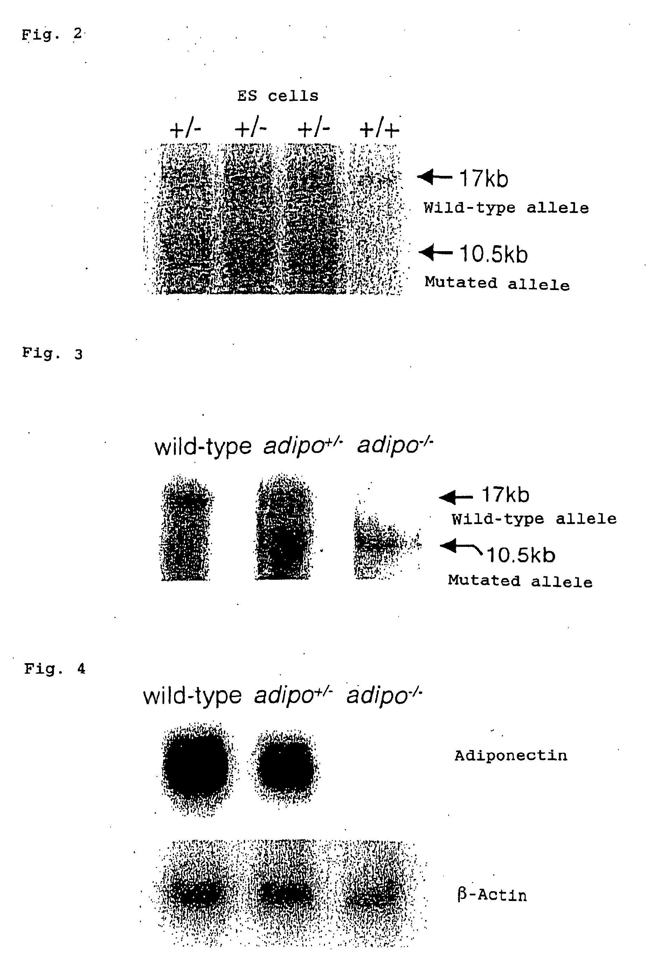

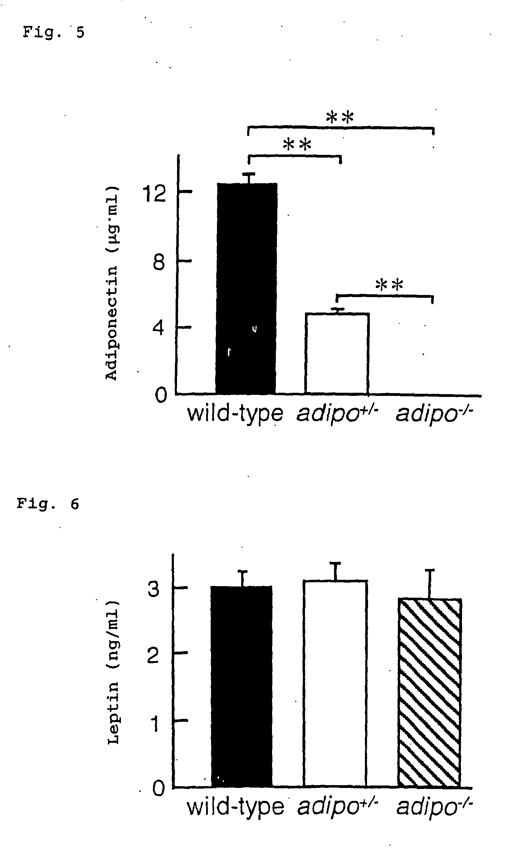

Specifically, the adiponectin gene knockout mice were generated through homologous recombination as shown in Fig. 1. In order to knockout the mouse adiponectin gene, a targeting vector was constructed by substituting a neo resistance gene for exons 2 and 3 encoding adiponectin. Southern blotting confirmed 5 clones of mutually independent homologous recombination (Fig. 2). Chimeric mice were generated from ES cells having a 129/Sv background and crossbred with BI/6, to thereby produce heterozygous knockout mice. The genotype of the mice was checked by Southern blotting (Fig. 3).

### (2) Insulin tolerance test

Test mice were fasted but only during the insulin tolerance test. Human insulin was intraperitoneally administered to each mouse at a dose of 0.7 mU per gram body weight. Blood samples were drawn from the tail vein, and blood sugar levels were measured by means of a Glutest-Ace (registered trademark, product of Sanwa Kagaku Kenkyusho Co., Ltd.).

### (3) Glucose tolerance test

Before starting the test, test mice were fasted at least 16 hours, and afterwards, glucose was perorally administered at a dose of 1.5 mg per gram body weight. Blood samples were drawn from ocular fundus veins, and blood sugar levels and insulin levels were determined by means of a Glutest-Ace (registered trademark, product of Sanwa Kagaku Kenkyusho Co., Ltd.) and a rat insulin RIA kit (Product of Amersham Pharmacia Biotech, Inc.), respectively.

### (4) Measurement of blood lipid levels

Following fasting for 16 hours, levels in blood of free fatty acid, neutral fat, and total cholesterol were measured by means of a NEFAC-test, a TGL-type, and a Tchol E-type (products of Wako), respectively.

### (5) Measurement of blood leptin and adiponectin

Following fasting for 16 hours, levels in blood of leptin and adiponectin were measured by means of a Quintikine M kit (product of R&D) and an adiponectin RIA kit (product of LINCO), respectively.

### (6) Creation of a vascular intimal thickening model by placement of a cuff

A 2.0-mm polyethylene tube (PE-50) was placed in the femoral artery. Two weeks thereafter, the artery was formalin-fixed with pressure, and removed together with the opposite-side, uncuffed artery, which served as a control artery. Each of the thus-removed blood vessels was sliced to obtain continuous ring-shaped specimens, each having a length of 10 mm. Ten slices were taken and subjected to HE staining. The inner diameter of the blood vessel, the thickness of the intima, and the thickness of the media were measured, and intima/media ratio was calculated.

### B. Results

### (1) Mouse adiponectin gene knockout mice

Through Northern blotting of white adipose tissue, expression levels of adiponectin in the heterozygous (adipo +/-) knockout mice were found to be reduced by about 60%, and the homozygous (adipo -/-) knockout mice were found to exhibit completely no adiponectin expression (Fig. 4). Indeed, when blood adiponectin level was measured in the heterozygous (adipo +/-) knockout mice, the magnitude of reduction was found to be about 60%, and the levels in the heterozygous (adipo +/-) knockout mice were found to be lower than the detectable level (Fig. 5). With respect to the blood leptin level, no difference was observed (Fig. 6).

### (2) Insulin resistance of mouse adiponectin gene knockout mice

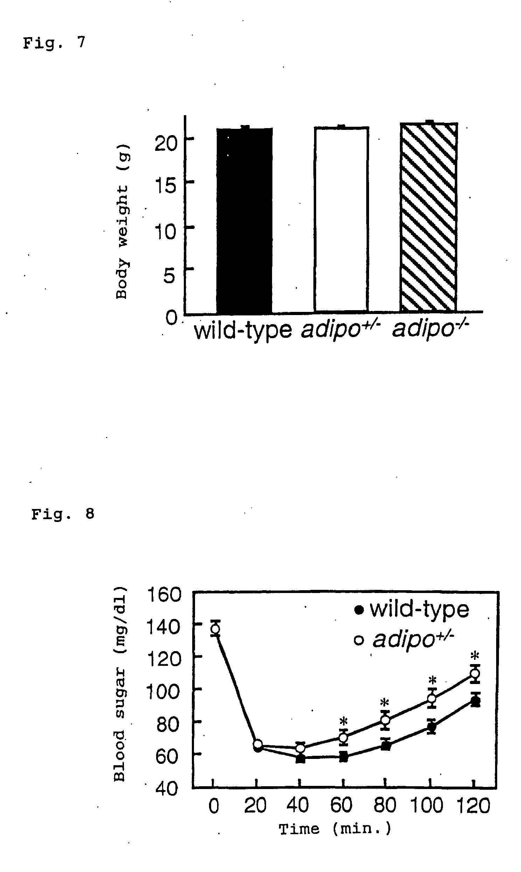

In three groups of 6-week-old mice; i.e., wild-type group, heterozygous (adipo +/-) knockout group, and homozygous (adipo -/-) knockout group, no difference was observed in terms of body weight (Fig. 7). Six-week-old wild-type mice and heterozygous (adipo +/-) knockout mice were subjected to an insulin tolerance test, to thereby check their insulin sensitivity. The degree of reduction in blood sugar level in response to exogenous insulin was statistically significantly low in the heterozygous (adipo +/-) knockout mice, proving that the heterozygous knockout mice had insulin resistance (Fig. 8).

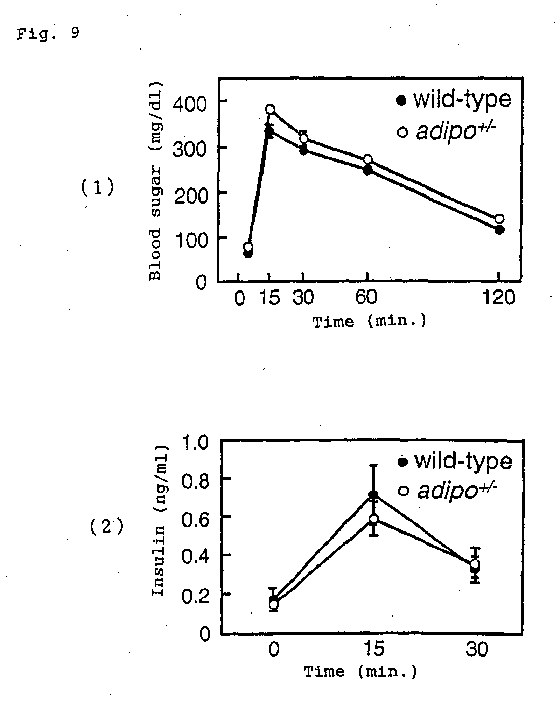

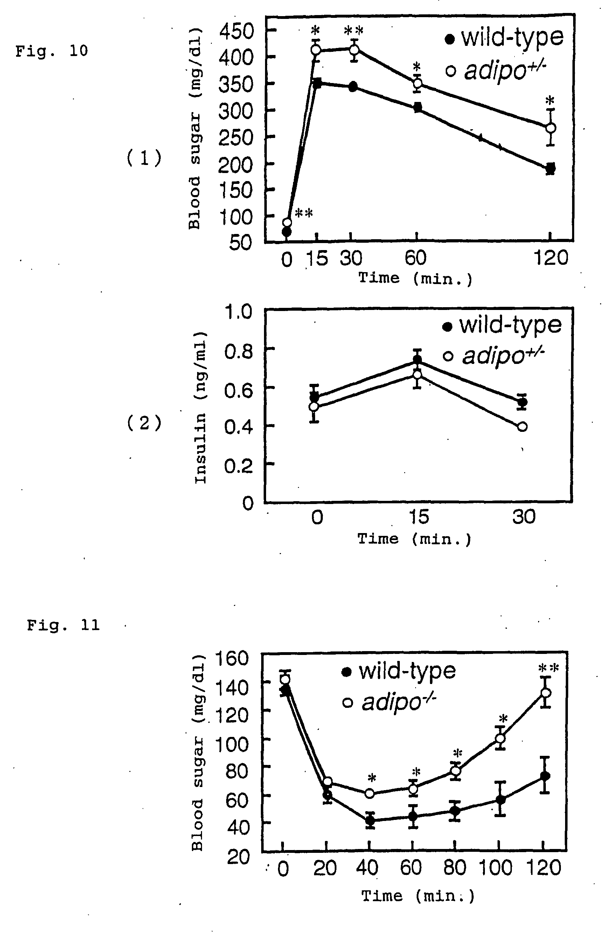

Next, a glucose tolerance test was performed. No difference was observed between the two groups of wild-type mice and heterozygous (adipo +/-) knockout mice in terms of blood sugar or insulin level (Fig. 9). However, as compared with the wild-type mice, the heterozygous (adipo +/-) knockout mice, after having been loaded with 10-week high fat diet, exhibited a significantly high blood sugar level before and after loading with glucose, though the body weight remained in a similar level (Fig. 10).

Subsequently, analysis on the homozygous (adipo -/-) knockout mice was performed.

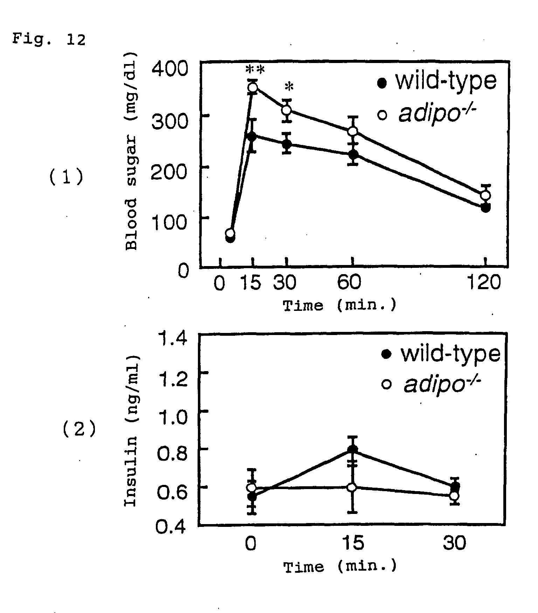

An insulin tolerance test performed on 6-week-old wild-type mice and homozygous (adipo -/-) knockout mice. As compared with the wild-type mice or the heterozygous (adipo +/-) knockout mice, the degree of reduction in blood sugar level in response to exogenous insulin was statistically significantly low in wild-type or heterozygous (adipo +/-) knockout mice, proving that the homozygous (adipo -/-) knockout mice had insulin resistance higher than the corresponding levels of the wild-type mice and heterozygous (adipo +/-) knockout mice (Fig. 11).

Next, a glucose tolerance test was performed. In both stages of during fasting and after glucose loading, the homozygous (adipo -/-) knockout mice exhibited blood sugar levels higher than the case of the wild-type mice. This makes it clear that homozygous (adipo -/-) knockout mice had slightly impaired glucose tolerance in addition to insulin resistance (Fig. 12). Before administration and 30 minutes after administration, no difference was observed between the wild-type group and the homozygous (adipo -/-) knockout group in terms of the insulin levels before and after glucose loading. However, the homozygous (adipo -/-) knockout mice showed a somewhat low insulin level at 15 min (Fig. 12).

### (3) Blood neutral fat level in adiponectin homozygous (adipo -/-) knockout mice

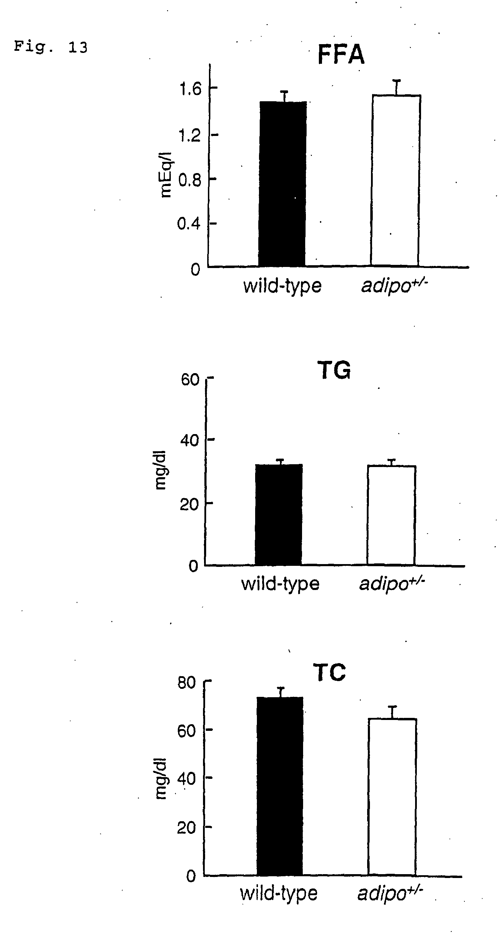

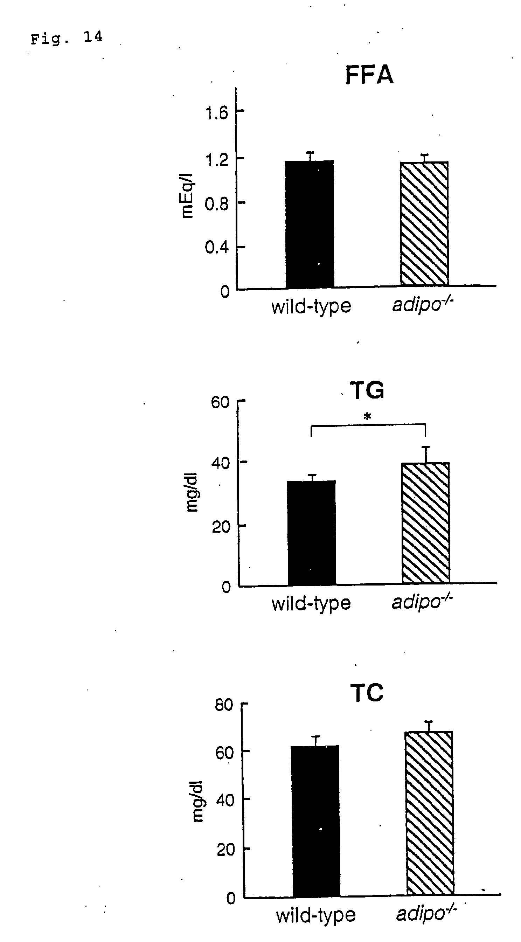

In order to check the effect of adiponectin on lipid metabolism, levels, in blood, of free fatty acid (FFA), neutral fat (TG), and total cholesterol (TC) were determined in wild-type, heterozygous (adipo +/-) knockout, and homozygous (adipo -/-) knockout mice (Figs. 13 and 14). The heterozygous (adipo +/-) knockout mice did not show any difference in level of any of the three test items as compared with the wild-type mice (Fig. 13). However, the homozygous (adipo -/-) knockout mice showed significantly higher blood neutral fat levels than the wild-type mice (Fig. 14) .

### (4) Thickening of the intima in cuff-injured models of mouse-adiponectin hetero-deficient mice

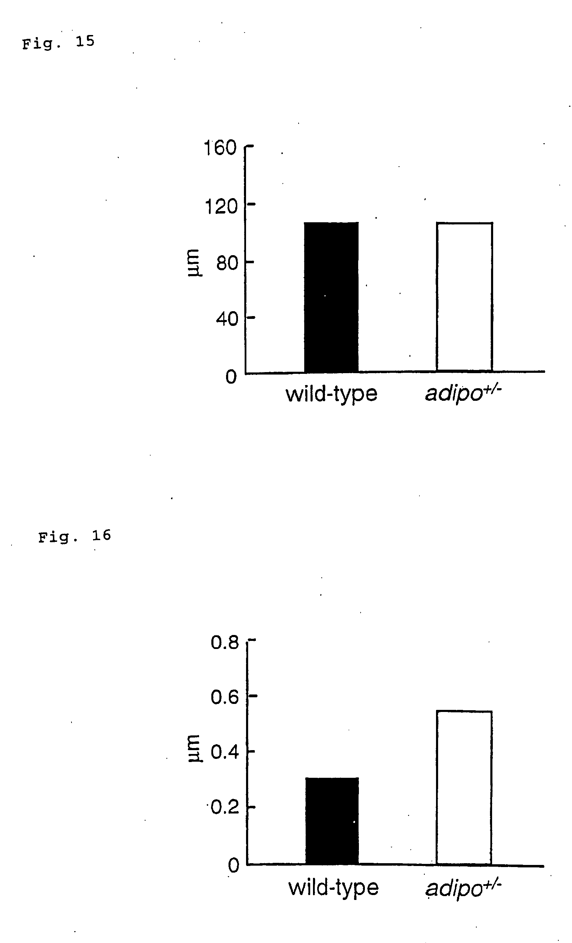

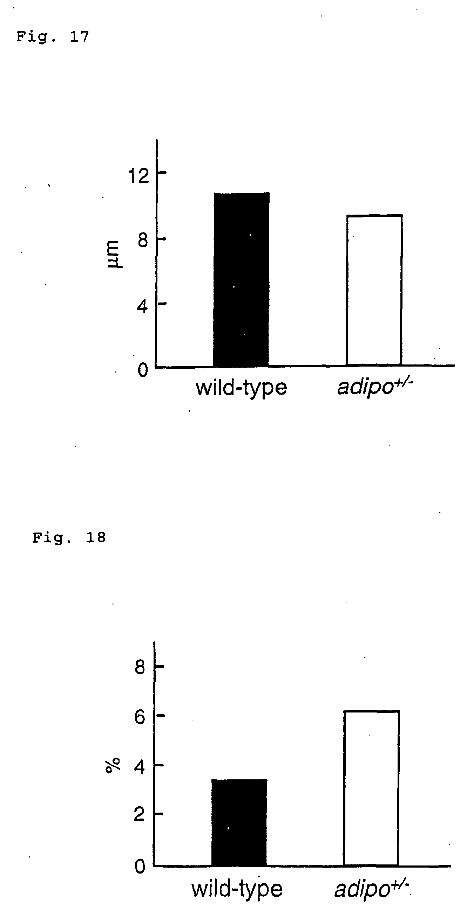

In order to investigate the effect of adiponectin on arteriosclerosis, the degree of intimal thickening induced by cuff placement was measured in the wild-type mice and the heterozygous (adipo +/-) knockout mice for comparison therebetween. No difference was observed between the two groups in terms of the vascular inner diameter after cuff-induced injury was created (Fig. 15). When 2 weeks had elapsed after creation of cuff injury, the heterozygous (adipo +/-) knockout mice showed about 1.8 times the thickness of the intima of the wild-type mice (Fig. 16). However, no difference was observed between the two groups in terms of the thickness of the media (Fig. 17). The intima/media ratio of the heterozygous (adipo +/-) group exhibited a ratio about two-fold that of the wild-type group (Fig. 18).

### Industrial Applicability

The animals of the present invention enable elucidation of the onset mechanisms of obesity, diabetes, and arteriosclerosis. Moreover, they are useful for screening preventive and therapeutic drugs for treating any of these pathological conditions.

## Claims

1. A non-human animal which is deficient in functions of adiponectin.

2. The non-human animal as recited in claim 1, wherein the animal is a mouse.

3. An animal model of obesity and/or diabetes, which comprises a non-human animal as recited in claim 1 or 2.

4. An arteriosclerosis animal model, which comprises a non-human animal as recited in claim 1 or 2.

5. A method of screening obesity and/or diabetes preventive and/or therapeutic agents, **characterized by** comprising administering a test drug to a non-human animal deficient in functions of adiponectin.

6. A method of screening arteriosclerosis preventive and/or therapeutic agent, **characterized by** comprising administering a test drug to a non-human animal deficient in functions of adiponectin.
